# EUROPÄISCHE PATENTSCHRIFT

(11) **EP 1 306 050 B1**
(45) Veröffentlichungstag und Bekanntmachung des Hinweises auf die Patenterteilung: **19.05.2004**
(21) Anmeldenummer: 01124379.7
(22) Anmeldetag: 24.10.2001
(51) Int. Cl.: A61B 5/04, A61B 5/042, A61B 19/00

(54) **Navigierte Mikrosonde**
Microprobe with navigation system
Microsonde avec système de navigation

(43) Veröffentlichungstag der Anmeldung: 02.05.2003
(73) Patentinhaber: BrainLAB AG, 85551 Kirchheim/Heimstetten (DE)
(72) Erfinder: Hartlep, Andreas Dr., 80803 München (DE)
(74) Vertreter: Schwabe - Sandmair - Marx

(56) Entgegenhaltungen:
- WO-A-01/49197
- WO-A-01/76497
- WO-A-98/39669
- US-A- 4 461 304
- US-A1- 2001 027 272

## Beschreibung

Die vorliegende Erfindung betrifft eine Vorrichtung zur elektrophysiologischen Lokalisierung von Zielgebieten im Gehirn gemäß dem Oberbegriff des Patentanspruchs 1.

Mit zunehmendem Altersdurchschnitt der Bevölkerung in den Industrienationen wächst auch das Auftreten altersbedingter Erkrankungen. Zu den häufigsten und am meisten belastenden Erkrankungen zählen dabei sicherlich das Parkinsonsche Syndrom und essentieller Tremor. Solche neurodegenerativen Erkrankungen werden, wenn medikamentöse Behandlungen keinen Erfolg mehr zeigen, durch hirnchirurgische Eingriffe behandelt, deren Erfolg stark von der korrekten Bestimmung des im Gehirn gelegenen Zielgebietes abhängig ist. Eine solche Lokalisation erfolgt seit einiger Zeit oftmals durch elektrophysiologische Ableitungen der neuronalen Entladungsmuster entlang einer stereotaktisch vorgegebenen Trajektorie. Dabei werden Mehrkanal-Mikrosonden verwendet, die mittels eines Manipulators in das Gehirn des Patienten eingebracht werden und die an ihrem aktiven Ende eine Vielzahl von dichtgepackten, axial aneinander gereihten Mikroelektroden aufweisen, über welche elektrophysiologische Ableitungen im Zielgebiet erhalten werden. Eine solchermaßen durchgeführte, exakte Zielbestimmung ist extrem wichtig, denn eine fehlplatzierte therapeutische Maßnahme kann mitunter gravierende Nebenwirkungen für den Patienten aufweisen. Beispielhaft sei hier die Durchführung einer Pallidotomie bei einem Parkinson-Patienten genannt, die aufgrund einer fehlplatzierten Koagulationselektrode eine irreversible Schädigung des in unmittelbarer Nähe zum Pallidum liegenden Tractus Opticus zur Folge hätte. Neben einer weiterhin bestehenden Parkinson-Symptomatik hätte der betreffende Patient dann zusätzlich eine unter Umständen starke Einschränkung seiner Sehleistung zu erleiden.

Mit Hilfe bisher bekannter Mikrosonden-Lokalisierungstechniken können zwar grundsätzlich funktionelle Bereich im Gehirn aufgefunden werden, jedoch benötigen selbst erfahrene Hirnchirurgen mehrere Vorgänge mit einer Testsonde, um ein einigermaßen korrektes Bild der Schichten des Aufbaus der Zielgebiete zu erhalten. Aus der Praxis ist aber auch bekannt, dass viele Hirnchirurgen wenig oder gar keine Erfahrung mit der Identifikation der Areale durch deren spezifische Aktivitäten haben. Eine solche traditionelle Vorgehensweise mit mehreren Sondeneinführungen dauert je nach Schwierigkeit zwischen 6 und 20 Stunden. Während eines Großteils dieser Zeit muss der Patient bei Bewußtsein sein, damit der Erfolg der elektrischen Stimulation anhand der Abwesenheit der Erkrankung kontrolliert werden kann. Einen weiteren Nachteil bilden natürlich die immer wieder notwendigen Penetrationen der Gehirnsubstanz.

Zum Stand der Technik betreffend Mikrosonden und medizinische Navigationssysteme (siehe unten) wird auf die folgenden Schriften verwiesen: US5,855,801; US5,843,148; US5,833,709; US5,800,535; US5,782,645; US5,755,759; US5,713,922; US5,524;619; US5,524,338; US5,496,369; US5,411,540; US5,388,577; US5,215,088; US4,969,468; US4,890,623; US4,837,049.

US 4, 461, 304 offenbart eine Mikrosonde gemäß dem Oberbegriff des Anspruchs 1.

Es ist die Aufgabe der vorliegenden Erfindung, eine Vorrichtung zur elektrophysiologischen Lokalisation von Zielgebieten im Gehirn bereitzustellen, welche die oben genannten Nachteile nicht mehr aufweist. Insbesondere soll eine hohe Präzision des Zielfindungsverfahren im Sub-Millimeterbereich erzielt werden, und es soll dem Operateur eine fundierte und verständliche Hilfestellung im Umgang mit den ermittelten Ableitungsdaten gegeben werden.

Diese Aufgabe wird erfindungsgemäß dadurch gelöst, dass der Mikrosonde eine Trackingeinrichtung zugeordnet wird, welche es gestattet, die Mikrosonde mittels eines Neuronavigationssystems positionell zu erfassen. Der Vorteil liegt hierbei insbesondere darin, dass mit einer solchen Vorrichtung die Planung aber auch die Durchführung der elektrophysiologischen Lokalisation mittels der Mikrosonde schon mit sehr viel höherer Anfangsgenauigkeit durchgeführt werden kann, und damit auch mit einer geringeren Anzahl an Einbringungsvorgängen und in sehr viel kürzerer Zeit. Die Anzahl der erforderlichen Trajektorien wird minimiert, wodurch auch dieser gesamte Lokalisierungsvorgang minimal invasiv wird. Die Neuronavigation bietet sowohl bei der Berechnung der Zielkoordinaten wie auch bei dem operativen Eingriff vielfältige Möglichkeiten. Die Lokalisation erfolgt zum einen über die physiologische Identifikation des Zielgebietes mittels der Mikrosonde, zum anderen über die anatomische Navigation.

Bei einer bevorzugten Ausführungsform der vorliegenden Erfindung weist das Navigationssystem eine Bildschirmausgabe auf, auf welcher die Auswertungen der elektrophysiologischen Lokalisation und der Navigationsdaten gemeinsam dargestellt werden. Die anfallenden elektrophysiologischen Daten (wie Ableitungen oder Spike-Frequenzen) können dabei zusammen mit anatomischen Bilddaten, die dem Navigationssystem zur Verfügung stehen, in einer einheitlichen Darstellung und unter Einbeziehung einer Datenbank für den Operateur dargestellt werden. Dadurch kann die Operationstechnik verbessert und das Risiko für den Patienten minimiert werden. Neuronavigationssysteme, wie sie zum Beispiel aus der DE 196 39 615 C2 bekannt sind, arbeiten mit Patientendaten aus vorab aufgenommenen Schichtbild-Erfassungsverfahren, zum Beispiel CT- oder MR-Aufhahmeverfahren. Über die Bildschirmausgabe können aus diesen Daten rekonstruierte Bilder zusammen mit Bildern der Mikrosonde selbst und deren Ergebnisdaten angezeigt werden, und der Operateur kann dann das Spitzenmuster der elektrophysiologischen Navigation, das eine Auflösung einzelner Neuronen bietet, mit den Bildinformationen und theoretischem Wissen vergleichen, um die Position der Sonde oder einer Mikroelektrode darauf exakt zu bestimmen. Die Erfindung kombiniert damit die beiden Lokalisierungsverfahren zu einer Navigation mit hoher Auflösung unter Verwendung einer bedienerfreundlichen und einfach verwendbaren Anwenderschnittstelle.

Gemäß einer vorteilhaften Ausführungsform hat die Mikrosonde 25 bis 32 Mikroelektroden. Wenn am aktiven Ende der Mikrosonde 32 dichtgepackte Mikroelektroden vorhanden sind, wird es möglich sein, ein Zielgebiet mit der Mikrosonde komplett und relativ schnell zu durchfahren und anschließend von jeder der 32 Mikroelektroden Nervensignale aus verschiedenen Hirnkernsegmenten des Zielgebiets simultan als neuronale Entladungsmuster abzuleiten.

Gemäß einer erfindungsgemäßen Ausgestaltung ist das Navigationssystem ein optisches Navigationssystem, wobei die Trackingeinrichtung an einem Manipulator für die Mikrosonde angebracht ist und aus einer Markeranordnung, insbesondere aus drei Reflexionsmarkern, besteht, deren räumliche Position von Kameras des Navigationssystems erfasst wird.

Andererseits ist es möglich, eine magnetische Navigation bereitzustellen, also ein Navigationssystem, das als Magnet-Navigationssystem ausgestaltet ist, wobei die Trackingeinrichtung an einem Manipulator für die Mikrosonde oder an der Mikrosonde selbst angebracht ist und aus einer Spulenanordnung, insbesondere aus zwei Miniaturspulen, besteht, deren räumliche Position in einem aufgebauten Magnetfeld erfasst wird.

In beiden oben genannten Fällen ist es von Vorteil, wenn das Navigationssystem ferner eine Patiententrackingeinrichtung umfasst, mittels der eine Momentanposition des Patientenkopfes in Echtzeit ermittelt wird, so dass Bewegungen des Patienten keinen störenden oder Ungenauigkeiten hervorrufenden Einfluss auf die Navigation bzw. Lokalisation haben.

Die beiden Lokalisierungssysteme, nämlich das elektrophysiologische Lokalisierungssystem mit der Mikrosonde und das Neuronavigationssystem können sich ergänzen und dabei synergistische Effekte bereitstellen. So ist die erfindungsgemäße Vorrichtung einerseits mit Vorteil so ausgestaltet, dass das Navigationssystem eine Computereinheit aufweist, welche die Daten aus dem elektrophysiologischen Ableitungen funktionaler Gehirnbereiche, den Sondenvorschub, sowie die Navigationsdaten miteinander verknüpft und die Navigation anhand von Positionsdaten und Informationen aus den elektrophysiologischen Ableitungen funktioneller Gehirnbereiche anpasst. Das Navigationssystem kann dabei von der sehr hohen Genauigkeit der elektrophysiologischen Lokalisation profitieren, und es kann anhand dieser genauen Informationen weitere anatomische Punkte sehr exakt zuordnen. Eine zweite Möglichkeit besteht darin, die erfindungsgemäße Vorrichtung so auszubilden, dass das Navigationssystem eine Computereinheit umfasst, welche die Daten aus den elektrophysiologischen Ableitungen funktionaler Bereiche des Gehirns sowie die Navigationsdaten miteinander verknüpft und Positionsdaten und Informationen aus den elektrophysiologischen Ableitungen funktioneller Gehirnbereiche in die dem Navigationssystem zur Verfügung stehenden Anatomiedaten einarbeitet. Hierdurch kann beispielsweise ein "virtueller Gehirnatlas" für den jeweiligen Patienten erstellt werden, der für spätere Behandlungen oder Diagnosen zur Verfügung steht.

Ganz allgemein macht die vorliegende Erfindung die Erfassung bestimmter funktionaler Bereiche im Gehirn möglich, und zwar entweder für die herkömmliche Chirurgie, für eine sogenannte Deep-Brain-Simulation (DBS) oder für neurologische Untersuchungen. Die Erfindung betrifft in diesem Sinne auch die hierin beschriebenen Verfahren mit gleichzeitigem Einsatz von Mehrkanal-Mikrosonden und einem Neuronavigationssystem, wobei insbesondere die Visualisierung und der Datenbankabgleich mit vorab erstellten Bilddaten aus Schichtbildaufnahmeverfahren den vorteilhaften Effekt der Kombination bewirken.

Die Erfindung wird im Weiteren anhand einer bevorzugten Ausführungsform näher erläutert. In den Zeichnungen zeigen:
- Figur 1: eine schematische Ansicht eines erfindungsgemäßen Navigations- und Lokalisationssystems;
- Figur 2: ein Beispiel für die Signalableitung an einer ins Gehirn eingesetzten Mikrosonde; und
- Figur 3: eine Möglichkeit, die mit einer erfindungsgemäßen Vorrichtung erhaltenen Signale auf einer Bildschirmausgabe darzustellen.

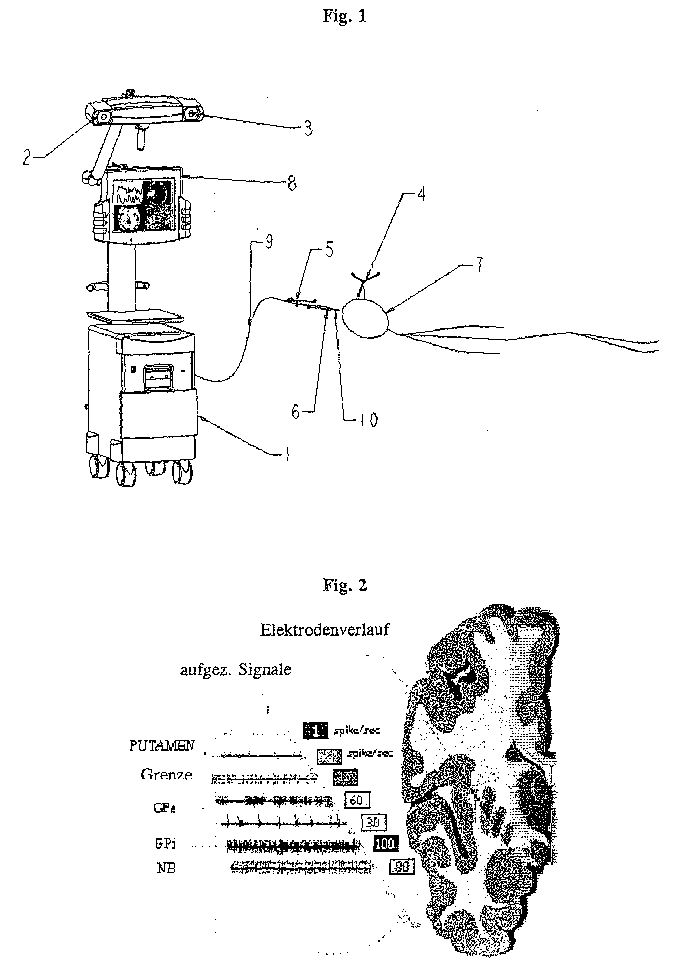

Die Figur 1 ist eine schematische Darstellung eines kombinierten Navigations- und Lokalisationssystems mit einer erfindungsgemäßen Vorrichtung. Links im Bild ist das Neuronavigationssystem dargestellt, welches als Hauptkomponenten den Navigationscomputer 1, den Bildschirm 8 sowie den Kamerahalter mit den Kameras 2 und 3 aufweist. Dieses optische Navigationssystem kann Markeranordnungen tracken, und damit auch die mit diesen Markeranordnungen versehenen Objekte. Im vorliegenden Fall sind diese Objekte eine Mikrosonde 10, die an einem Mikrosondenmanipulator 6 angeordnet ist, der wiederum eine Markeranordnung 5 trägt, die aus drei voneinander beabstandeten Markern besteht. Eine weitere Markeranordnung 4 ist am Kopf eines Patienten befestigt, wobei der Kopf in Figur 1 das Bezugszeichen 7 aufweist. Vom Mikrosondenmanipulator 9 geht ein Kabel 9 zum Navigationscomputer 1, wobei das Kabel 9 die Messergebnisse der Mikrosonde 10 in das Computersystem überträgt. Mit der auf ihm angeordneten Markeranordnung 5 kann der Mikrosondenmanipulator 6 und damit die Mikrosonde 10 im Erfassungsbereich der Kameras 2 und 3 getrackt, d. h. positionell verfolgt werden. Auf dem Bildschirm 8 kann dann die Position der Sonde in Relation zur Patientenanatomie dargestellt werden, wenn vorab von dem Kopfbereich des Patienten anatomische Daten mittels eines Schichtbildaufnahmeverfahren erfasst worden sind. Die Registrierung des Patienten und der Mikrosonde bzw. des Manipulators 5 erfolgt in bekannter Weise vor der Behandlung, und um Fehler durch Bewegungen des Patientenkopfes 7 auszuschließen, werden diese Bewegungen mittels der Markeranordnung 4 mit erfasst und in die Navigation eingearbeitet.

Die getrackte Mikrosonde 10 kann dann schon mit Unterstützung des Navigationssystems, also mit vorgegebener Trajektorie in den Kopf des Patienten eingeführt werden, um die Zielgebiete von Interesse, also die funktionalen Bereiche elektrophysiologisch zu lokalisieren. Dabei kann das Navigationssystem an der Bildschirmeinheit 8 eine Trajektorie vorschlagen und auch Abweichungen des tatsächlichen Verlaufs von dem geplanten Verlauf anzeigen, so dass schon beim ersten Einbringen eine relativ genaue Positionierung der Sonde möglich wird.

In Figur 2 ist ein Beispiel für einen Sondenverlauf an einem Gehirnschnittbild aufgezeigt. Wenn die Sonde 10 mit der dargestellten Trajektorie (Linie im Gehirnschnitt) eingebracht wird, können an den Mikroelektroden 11, die sich am aktiven Ende der Sonde befinden, Neuronensignale abgeleitet werden, die beispielhaft in vergrößerter Darstellung in der linken Bildhälfte in Figur 2 aufgezeigt sind.

Die Neuronavigation bietet sowohl bei der Berechnung der Zielkoordinaten wie auch beim operativen Eingriff vielfältige Möglichkeiten. Die Lokalisation erfolgt zum einen über die physiologische Identifikation des Zielgebietes mittels der Mikrosonde, zum anderen über die anatomische Navigation. Hier gibt es wiederum verschiedenen Optionen, die beliebig kombiniert werden können.

Zunächst kann anhand einer Linie, die durch zwei anatomische Strukturen (zum Beispiel commissura anterior et posterior) gelegt wird, geplant werden; diese Planung erfolgt mittels der im Neuronavigationssystem vorhandenen anatomischen Positionsdaten. Ferner kann man beispielsweise einen virtuellen Gehirnatlas über das Gehirn des Patienten legen, was ebenfalls mittels der Neuronavigationsplanung durchgeführt wird. Außerdem kann das Ziel in entsprechenden Strukturen direkt visualisiert werden, wenn beispielsweise durch geeignete MR-Bildgebungssequenzen des Gehirns entsprechende Bilder vorliegen. Diese Möglichkeiten ergeben in Kombination eine Genauigkeit der Zielgebietslokalisation im Millimeterbereich. Man kann nun mit diesen Koordinaten mit einem mechanischen Zielgerät, welches direkt in den Schädel des wachen Patienten geschraubt wird, die Elektrode im Gehirn platzieren. Mit der Neuronavigation lässt sich also mit einem sehr kleinen Aufsatz auf dem Schädel das Zielgebiet präzise anfahren und der OP-Vorgang minimal invasiv durchführen. Die bildgebundene Kontrolle des OP-Fortschrittes kann durch elektrophysiologische Messungen noch verbessert werden.

Die funktionelle Ableitung der Neuronenaktivität lässt entsprechend des Aufbaus des Gehirns Rückschlüsse auf die Position der Mikrosonde zu. Die Mikrosonde besteht dabei aus einer feinen Mikroelektrode mit einem Durchmesser von 0,1 mm im Inneren eines Hüllrohres, die unter elektrophysiologischer Ableitung immer näher an das Zielgebiet gebracht wird. Der Vorschub dieser Mikroelektrode wird laufend an das Navigationssystem übermittelt, so das mit hoher Genauigkeit die "z"-Koordinate der Sondenspitze gemessen und dargestellt werden kann. Die Zielregion verrät sich dann nicht nur durch die errechnete Position auf den Aufnahmen, sondern auch durch die spezifischen Aktivitätsmuster mit hoher Präzision.

Anschließend kann diese Testsonde wieder in ein Hüllrohr zurückgezogen und das unisolierte Ende des Hüllrohrs als Stimulationssonde benutzt werden. Die Stimulation durch schwache Stromreizung ist einerseits der positive Beweis, das richtige inhibierende Areal identifiziert zu haben, andererseits aber auch nötig, um nicht zum Beispiel in zu große Nähe des Sehnervs zu kommen. Sobald die scheinbar korrekten Koordinaten im Hirn des Patienten identifiziert sind, wird eine neue Elektrode eingefügt, und das Zielgebiet wird bis zu einem Durchmesser von mehreren Millimetern zerkocht.

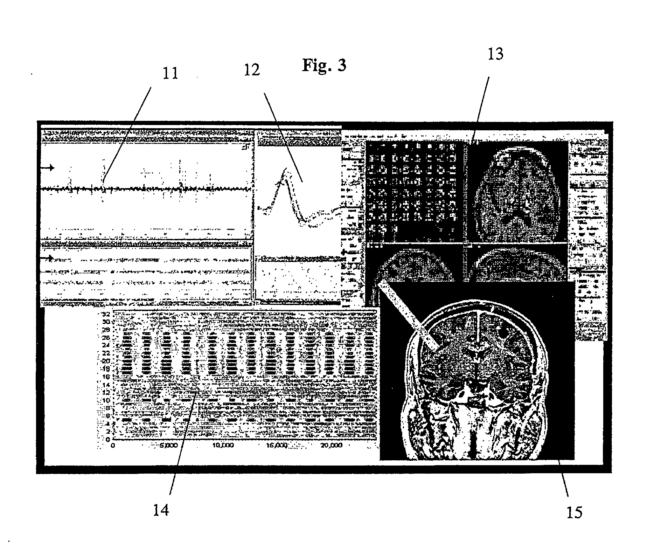

Während der gesamten Behandlung kann der Operateur durch die Darstellung der Navigations- und Lokalisationsergebnisse auf der Bildschirmausgabe unterstützt werden. Ein Beispiel für eine solche Bildschirmausgabe ist in Figur 3 gezeigt. Dabei ist der Bildschirm des Navigationssystems in mehrere geöffnete Fenster unterteilt, so dass dem Operateur die für ihn interessanten Informationen auch gleichzeitig zur Verfügung stehen.

Im vorliegenden Fall zeigt die Bildschirmansicht in Figur 3 ein Spike-Template 12, eine Einzelkanaldarstellung 11, eine Navigationskarte 13, eine Zielkarte 15 mit Trajektorie sowie eine 32-Kanalableitung 14. Mit Hilfe dieser kombinierten Bildausgabe ist es - wie oben schon angedeutet - für den Operateur möglich, seinen Eingriff sorgfältig zu planen und angeleitet durchzuführen, und zwar mittels einer topographischen Zuordnung der im Zentralnervensystem abgeleiteten neuronalen Entladungsmuster durch eine *µ*m-genaue Subparziellierung der verschiedenen basalen Kerne bzw. der funktionell und anatomisch abgrenzbaren Segmente im Sinne einer Mikrosomatotopie. Neben der Erhöhung der Ortungspräzision des Zielgebietes wird mit einem solchen integrierten Navigations- und Lokalisationssystem auch eine drastische Verkürzung der Operationsdauer erzielt. Dies hat neben einer geringeren gesundheitlichen Belastungen für den Patienten auch eine geringere Kostenbelastung zur Folge.

## Patentansprüche

1. Vorrichtung zur elektrophysiologischen Lokalisation von Zielgebieten im Gehirn, mit einer Mehrkanal-Mikrosonde (10), die an ihrem aktiven Ende eine Vielzahl von dicht gepackten, axial aneinandergereihten Mikroelektroden aufweist, über welche elektrophysiologische Ableitungen im Zielgebiet erhalten und an eine Auswertungseinheit weitergeleitet werden, **dadurch gekennzeichnet, dass** der Mikrosonde (10) eine Trackingeinrichtung (5) zugeordnet ist, welche es gestattet, die Mikrosonde (10) mittels eines Neuronavigationssystems (1, 2, 3, 8) positionell zu erfassen und das Einbringen der Sonde stereotaktisch zu planen und dass das Navigationssystem eine Computereinheit (1) aufweist, welche die Daten aus den elektrophysiologischen Ableitungen funktionaler Bereiche des Gehirns sowie die Navigationsdaten miteinander verknüpft und die Navigation anhand von Positionsdaten und Informationen aus den elektrophysiologischen Ableitungen funktioneller Gehirnbereiche anpasst.

2. Vorrichtung nach Anspruch 1, **dadurch gekennzeichnet, dass** das Navigationssystem eine Bildschirmausgabe (8) aufweist, auf welcher die Auswertungen der elektrophysiologischen Lokalisation und der Navigationsdaten gemeinsam dargestellt werden.

3. Vorrichtung nach einem der Ansprüche 1 oder 2, **dadurch gekennzeichnet, dass** die Mikrosonde (10) 25 bis 32, Mikroelektroden aufweist.

4. Vorrichtung nach einem der Ansprüche 1 bis 3, **dadurch gekennzeichnet, dass** das Navigationssystem ein optisches Navigationssystem ist, wobei die Trackingeinrichtung an einem Manipulator (6) für die Mikrosonde (10) angebracht ist und aus einer Markeranordnung (5), insbesondere aus drei Reflexionsmarkern, besteht, deren räumliche Position von Kameras (2, 3) des Navigationssystems erfasst wird.

5. Vorrichtung nach einem der Ansprüche 1 bis 3, **dadurch gekennzeichnet, dass** das Navigationssystem ein Magnetnavigationssystem ist, wobei die Trackingeinrichtung an einem Manipulator (6) für die Mikrosonde (10) oder an der Mikrosonde selbst angebracht ist und aus einer Spulenanordnung, insbesondere aus zwei Miniaturspulen, besteht, deren räumliche Position in einem aufgebauten Magnetfeld erfasst wird.

6. Vorrichtung nach Anspruch 4 oder 5, **dadurch gekennzeichnet, dass** das Navigationssystem ferner eine Patiententrackingeinrichtung (4) umfasst, mittels der eine Momentanposition des Patientenkopfes in Echtzeit ermittelt wird.

7. Vorrichtung nach einem der Ansprüche 1 bis 6, **dadurch gekennzeichnet, dass** das Navigationssystem eine Computereinheit (1) aufweist, welche die Daten aus den elektrophysiologischen Ableitungen funktionaler Bereiche des Gehirns sowie die Navigationsdaten miteinander verknüpft und Positionsdaten und Informationen aus den elektrophysiologischen Ableitungen funktioneller Gehirnbereiche in die dem Navigationssystem zur Verfügung stehenden Anatomiedaten einarbeitet, insbesondere zur Erstellung eines Gehirnatlasses.

## Claims

1. A device for electrophysiologically localising target areas in the brain, comprising a multi-channel microprobe (10) which at its active end comprises a multitude of tightly packed microelectrodes arranged axially in rows, via which electrophysiological efferences are obtained in the target area and forwarded to an evaluating unit, **characterised in that** said microprobe (10) is assigned to a tracking device (5) which allows said microprobe (10) to be positionally detected by means of a neuronavigation system (1, 2, 3, 8) and the insertion of said probe to be stereotactically planned, and **in that** said navigation system comprises a computer unit (1) which links the data from the electrophysiological efferences of functional areas of the brain and the navigational data with one another and adapts navigation by way of positional data and information from the electrophysiological efferences of functional areas of the brain.

2. The device as set forth in claim 1, **characterised in that** said navigation system comprises a screen output (8) on which the evaluations of electrophysiological localisation and of the navigational data are shown together.

3. The device as set forth in any one of claims 1 or 2, **characterised in that** said microprobe (10) comprises 25 to 32 microelectrodes.

4. The device as set forth in any one of claims 1 to 3, **characterised in that** said navigation system is an optical navigation system, wherein said tracking device (5) is attached to a manipulator (6) for said microprobe (10) and consists of an arrangement of markers (5), in particular of three reflection markers, whose spatial position is detected by cameras (2, 3) of said navigation system.

5. The device as set forth in any one of claims 1 to 3, **characterised in that** said navigation system is a magnetic navigation system, wherein said tracking device is attached to a manipulator (6) for said microprobe (10) or to said microprobe itself and consists of an arrangement of coils, in particular of two miniature coils, whose spatial position is detected in an established magnetic field.

6. The device as set forth in claim 4 or 5, **characterised in that** said navigation system further comprises a patient tracking device (4), by means of which a current position of the patient's head is detected in real time.

7. The device as set forth in any one of claims 1 to 6, **characterised in that** said navigation system comprises a computer unit which links the data from the electrophysiological efferences of functional areas of the brain and the navigational data with each other and works positional data and information from the electrophysiological efferences of functional areas of the brain into the anatomical data available to the navigation system, in particular for producing a brain atlas.

## Revendications

1. Dispositif de localisation électrophysiologique de zones cibles dans le cerveau, avec une microsonde à plusieurs canaux (10) qui présente à son extrémité active un grand nombre de microélectrodes alignées axialement en rangs serrés, via lesquelles on obtient des dérivations électrophysiologiques dans la zone cible et transmet celles-ci à une unité d'évaluation, **caractérisé en ce que** la microsonde (10) est affectée à un dispositif de suivi (5) qui permet de détecter la position de la microsonde (10) à l'aide d'un système de neuronavigation (1, 2, 3, 8) et de planifier l'introduction de la sonde par stéréotaxie et **en ce que** le système de navigation possède une unité informatique (1), laquelle combine ensemble des données des dérivations électrophysiologiques de zones fonctionnelles du cerveau ainsi que les données de navigation et adapte la navigation sur base de données de position et d'informations des dérivations électrophysiologiques de zones fonctionnelles du cerveau.

2. Dispositif suivant la revendication 1, **caractérisé en ce que** le système de navigation possède un écran d'affichage (8), sur lequel les résultats de la localisation électrophysiologique et des données de navigation sont représentés en commun.

3. Dispositif suivant l'une des revendications 1 ou 2, **caractérisé en ce que** la microsonde (10) présente 25 à 32 microélectrodes.

4. Dispositif suivant l'une des revendications 1 à 3, **caractérisé en ce que** le système de navigation est un système de navigation optique, le dispositif de suivi étant fixé à un manipulateur (6) pour la microsonde (10) et constitué d'un dispositif marqueur (5), en particulier de trois marqueurs à réflexion, dont la position spatiale est détectée par des caméras (2, 3) du système de navigation.

5. Dispositif suivant l'une des revendications 1 à 3, **caractérisé en ce que** le système de navigation est un système de navigation magnétique, le dispositif de suivi étant fixé à un manipulateur (6) pour la microsonde (10) ou sur la microsonde elle-même et constitué d'un dispositif à bobine, en particulier de deux bobines miniatures, dont la position spatiale est détectée dans un champ magnétique établi.

6. Dispositif suivant la revendication 4 ou 5, **caractérisé en ce que** le système de navigation comprend en outre un dispositif de suivi du patient (4), à l'aide duquel une position momentanée de la tête du patient est déterminée en temps réel.

7. Dispositif suivant l'une des revendications 1 à 6, **caractérisé en ce que** le système de navigation comprend une unité informatique (1) qui combine ensemble les données des dérivations électrophysiologiques de zones fonctionnelles du cerveau ainsi que les données de navigation et intègre des données de position et des informations provenant des dérivations électrophysiologiques de zones fonctionnelles du cerveau dans les données anatomiques dont dispose le système de navigation, en particulier pour l'élaboration d'un atlas du cerveau.
